# EUROPÄISCHE PATENTSCHRIFT

(11) **EP 0 452 623 B1**
(45) Veröffentlichungstag und Bekanntmachung des Hinweises auf die Patenterteilung: **02.11.1994**
(21) Anmeldenummer: 91101590.7
(22) Anmeldetag: 06.02.1991
(51) Int. Cl.: A61B 17/58, A61B 17/28

(54) **Vorrichtung zum Spannen chirurgischer Schraubverbindungen**
Device for bending surgical screw connections
Dispositif pour tendre les liaisons chirurgicales par vis

(30) Priorität: 05.04.1990 DE 4010977
(43) Veröffentlichungstag der Anmeldung: 23.10.1991
(73) Patentinhaber: AESCULAP AG, D-78501 Tuttlingen (DE)
(72) Erfinder: Eitenmüller, Jürgen, W-5024 Brauweiler (DE); Pommer, Axel, W-4630 Bochum (DE)
(74) Vertreter: Hoeger, Stellrecht & Partner

(56) Entgegenhaltungen:
- DE-U- 8 400 640
- DE-U- 8 813 344
- GB-A- 2 209 948

## Beschreibung

Die Erfindung betrifft eine Vorrichtung zum Spannen chirurgischer Schraubverbindungen.

Zur Fixierung von Knochenplatten und anderen Implantaten an Knochen, beispielsweise zur Festlegung von Knochenfragmenten nach Brüchen, ist es bekannt, Knochenschrauben zu verwenden, die nach Art bekannter Schrauben einen Kopf aufweisen und mittels eines Schraubwerkzeuges in den Knochen fest eingeschraubt werden.

Während dieses Vorgehen bei herkömmlichen Metallschrauben erfolgreich gewesen ist, haben sich bei Knochenschrauben aus resorbierbaren Kunststoffmaterialien Schwierigkeiten ergeben, da diese Kunststoffmaterialien eine geringere Festigkeit aufweisen als die herkömmlich verwendeten Metallschrauben.

Es ist bereits vorgeschlagen worden, bei Verwendung von resorbierbaren Kunststoffen einfache mit einem Außengewinde versehene Gewindestäbe in vorbereitete Gewinde an den zu fixierenden Knochenfragmenten einzuschrauben und die daraufgelegten Knochenplatten etc. dadurch mit dem Knochen zu verspannen, daß auf die aus Kunststoff bestehenden Gewindestäbe Muttern aufgeschraubt werden. Dabei sollen die Gewindestäbe beim Aufschrauben der Muttern unter Zugbeanspruchung gesetzt werden, so daß eine geringfügige Dehnung der Knochenschrauben eintritt. Die Muttern werden nur locker bis handfest auf den Gewindestab aufgeschraubt. Sobald man die Spannung der Gewindestäbe nachläßt, werden die Muttern dann fest gegen die Oberfläche der Knochenplatte etc. gezogen, so daß eine Spannung der Schraubverbindung eintritt.

Es ist Aufgabe der Erfindung, eine Vorrichtung anzugeben, mit welcher eine solche für Knochenschrauben aus resorbierbarem Kunststoffmaterial besonders geeignete Schraubfixierung hergestellt werden kann.

Diese Aufgabe wird erfindungsgemäß gelöst durch eine Vorrichtung zum Spannen chirurgischer Schraubverbindungen, bei der auf einen eingeschraubten Gewindestab während dessen Zugbeanspruchung eine Mutter aufschraubbar ist, die gekennzeichnet ist durch einen zentralen Zugstab für den Gewindestab, ein diesen Zugstab koaxial umgebendes, in Längsrichtung gegenüber diesem verschieblich und um die Längsachse frei drehbar gehaltenes Schraubelement für die auf den Gewindestab aufgeschraubte Mutter, durch ein das Schraubelement umgebendes, eine untere Abstützfläche aufweisendes Stützelement, an dem eine Spannvorrichtung gehalten ist, die über Umlenkelemente an dem Zugstab angreift und diesen von der Abstützfläche nach oben zieht.

Es ergibt sich durch die Anordnung von drei konzentrisch zueinander angeordneten Teilen eine besonders kompakte Vorrichtung, die geeignet ist, nicht nur einen in einen Knochen eingeschraubten Gewindestab in der gewünschten Weise unter Zugbeanspruchung zu setzen, sondern mit der gleichzeitig die bereits auf den Gewindestab aufgeschraubte Mutter in die Anlageposition an der Knochenplatte gebracht werden kann.

Dabei ist es besonders vorteilhaft, wenn bei einer bevorzugten Ausführungsform der zentrale Zugstab ein auf den Gewindestab passendes Innengewinde aufweist, mit dessen Hilfe eine zugfeste Verbindung zwischen Zugstab und Gewindestab herstellbar ist. Es sind aber auch andere lösbare Verbindungen möglich, die eine Übertragung von Zugkräften ermöglichen.

Es kann gemäß einer bevorzugten Ausführungsform vorgesehen sein, daß das Schraubelement ein den Zugstab umgebendes Rohr ist. Dieses Schraubelement kann formschlüssig in Ausnehmungen in der Mutter passende Vorsprünge tragen, es wäre auch möglich, daß das rohrförmig ausgebildete Schraubelement nach Art eines Schraubenschlüssels eine unrund ausgebildete Mutter formschlüssig umgreift.

Weiterhin ist es von Vorteil, wenn auch das Stützelement ein das Schraubelement konzentrisch umgebendes Rohr ist, dessen Abstützfläche vorzugsweise eine Zahnung trägt, so daß ein Abrutschen an der Abstützfläche auf der Knochenplatte beziehungsweise dem Knochen vermieden werden kann.

Bei einer bevorzugten Ausführungsform ist vorgesehen, daß die Spannvorrichtung zwei gegeneinander verschwenkbare Griffe trägt, von denen einer über eine Untersetzung seine Bewegung auf den Zugstab überträgt. Durch die Untersetzung ist es für den Arzt möglich, sehr große Zugspannungen auf den Gewindestab auszuüben, beispielsweise Zugspannungen in der Größenordnung von 90% der maximal zulässigen Zugspannung. Es genügt dann eine leichte Anlage der Mutter an der Anlagefläche, um nach dem Loslassen der Schraube doch eine kräftige Spannung der Schraubverbindung zu gewährleisten. Dabei ist es vorteilhaft, wenn man die Mutter nur ganz leicht andreht oder maximal mit einem Drehmoment in der Größenordnung bis 10 cm kp, um eine starke zusätzliche Spannung der Schraube, die bereits vorgespannt ist, zu vermeiden. Zu diesem Zweck kann eine Drehmomentbegrenzung in dem Schraubelement vorgesehen sein, so daß der Operateur die Mutter mittels des Schraubelementes spannen kann, ohne darauf achten zu müssen, daß keine zu große Spannung entsteht.

Die Spannvorrichtung kann bei einem bevorzugten Ausführungsbeispiel einen Freilauf für ein die Spannvorrichtung betätigendes Handelement und eine Arretierung für den Zugstab oder mit diesem verbundene Umlenkelemente aufweisen. Dadurch wird es möglich, die Bewegung des Handelementes wiederholt auszunutzen, um eine zusätzliche Spannung des Zugstabes zu erreichen. Ein solcher Freilauf kann beispielsweise in der Art ausgebildet sein, wie es bei Ausbringvorrichtungen für Silikondichtmassen bekannt ist, es können aber auch andere dem Fachmann an sich bekannte mechanische Vorrichtungen verwendet werden, beispielsweise Zahnradgetriebe mit Ratschensperren.

Bei einem bevorzugten Ausführungsbeispiel ist auf dem Zugstab oder einem mit dem Zugstab verbundenen Zugelement eine Spannmutter aufgeschraubt, die an der Vorrichtung frei drehbar gelagert ist und sich an dieser in axialer Richtung abstützt. Das Spannen des Zugstabes erfolgt dadurch einfach durch Verdrehung dieser Spannmutter. Diese kann vorzugsweise eine Flügelmutter sein.

Vorteilhaft ist es auch, wenn die Spannmutter gemäß einem bevorzugten Ausführungsbeispiel gegenüber der Vorrichtung mittels eines Kugellagers gelagert ist, so daß auch bei hoher Spannung eine leichte Verdrehbarkeit dieser Spannmutter gewährleistet ist.

Bei einem bevorzugten Ausführungsbeispiel greift am Zugstab unmittelbar oder mittelbar eine Zugfeder an, die mit einem von der Spannvorrichtung verschiebbaren Zugelement verbunden ist. Dadurch ergibt sich eine Übersetzung des Verschiebeweges der Spannvorrichtung und des Verschiebeweges des Zugstabes, das heißt die Zunahme der Spannung auf den Gewindestab kann feiner dosiert werden.

Dabei ist es günstig, wenn das Zugelement sich an dem Zugstab oder einem daran axial festgelegten Teil mittels eines Abstandshalters abstützt, welcher so lang ist, daß die Zugfeder gespannt ist. Solange die Spannung des Gewindestabes geringer ist als die Spannung der Zugfeder, bleibt der Abstandshalter in der Anlage, das heißt der Verschiebeweg der Spannvorrichtung entspricht dann dem Verschiebeweg des Gewindestabes. Für das anfängliche Einsetzen und für das anfängliche Spannen ist dies von Vorteil. Sobald die Spannung des Gewindestabes die Zugfederspannung übersteigt, wird die Zugfeder gedehnt, so daß sich die beschriebene Bewegungsübersetzung einstellt, das heißt eine größere Wegänderung der Spannvorrichtung führt nur zu einer relativ geringen Längung des Gewindestabes, wobei die Zugkraft auf den Gewindestab entsprechend der Längung der Zugfeder zunimmt.

Die Länge des Abstandshalters ist bei einem bevorzugten Ausführungsbeispiel vorzugsweise einstellbar, so daß der Übergang aus der unübersetzten Dehnung in die übersetzte Dehnung frei wählbar ist.

Das Abstützelement kann in ein die Vorrichtung umgebendes Gehäuse übergehen.

Vorzugsweise befindet sich gemäß einem bevorzugten Ausführungsbeispiel in diesem mindestens eine Öffnung zum Verdrehen des Schraubelementes. Das Schraubelement kann im Bereich der Öffnung gerändelt sein.

Günstig ist es auch, wenn bei einem bevorzugten Ausführungsbeispiel der Zugstab frei um seine Längsachse drehbar ist und an seinem der Verbindungsstelle zum Gewindestab abgewandten Ende einen Drehgriff trägt. Durch diese Ausgestaltung ist es möglich, bei festgehaltener Vorrichtung allein durch Verdrehung des Zugstabes den in den Zugstab eingesetzten Gewindestab in die im Knochen vorbereitete Öffnung einzuschrauben.

Dabei kann am Zugstab ein Mitnehmer frei drehbar und in axialer Richtung gar nicht oder beschränkt verschiebbar gelagert sein, an dem die Spannvorrichtung angreift. Die Spannvorrichtung selbst kann also ortsfest bleiben, obwohl der Zugstab verdrehbar ist.

Bei einem besonders bevorzugten Ausführungsbeispiel ist vorgesehen, daß in dem Zugstab, der Spannvorrichtung oder den Umlenkelementen Druck- beziehungsweise Zugsensoren angeordnet sind, die eine Anzeige betätigen. Der Operateur kann an dieser Anzeige die Spannung ablesen, die auf den Gewindestab ausgeübt wird, so daß er bei der Spannung bis dicht an die Abreißgrenze herangehen kann, gleichzeitig aber sicher ist, den Gewindestab nicht zu zerstören.

Vorteilhaft ist es dabei, wenn die Anzeige erst ab einem festgelegten Minimalwert betätigbar ist, so daß der Operateur erst im kritischen Bereich auf die Anzeige achten muß, bis dahin aber in seiner Konzentration ungestört sein kann.

Die nachfolgende Beschreibung einer bevorzugten Ausführungsform der Erfindung dient im Zusammenhang mit der Zeichnung der näheren Erläuterung. Es zeigen:
- Figur 1:: eine schematische Längsschnittansicht eines ersten Ausführungsbeispiels einer Spann- und Schraubvorrichtung für einen Gewindestab;
- Figur 2:: eine vergrößerte Teilansicht des Einschraubbereiches in Figur 1;
- Figur 3:: eine perspektivische Ansicht eines weiteren Ausführungsbeispiels einer Spann- und Schraubvorrichtung für einen Gewindestab und
- Figur 4:: eine Schnittansicht längs Linie 4-4 in Figur 3.

In der Zeichnung wird die Spannvorrichtung am Beispiel eines Röhrenknochens 1 dargestellt, an dem eine Knochenplatte 2 durch eine Schraubverbindung festgespannt werden soll.

Zu diesem Zweck wird in den Knochen 1 eine Gewindebohrung 3 eingebracht, die sich bis in die gegenüberliegende Knochenwand fortsetzen kann. In diese Gewindebohrung 3 wird ein Außengewindestab 4 aus einem resorbierbaren Kunststoffmaterial eingeschraubt, der beispielsweise aus Homopolymeren oder Copolymeren auf der Basis von L-Lactid, D-Lactid, D,L-Lactid, meso-Lactid oder Glycolid bestehen kann. Der Gewindestab 4 ragt weit aus dem Knochen 1 hervor.

Die Knochenplatte 2 wird anschließend auf den Knochen so aufgelegt, daß der Gewindestab 4 durch die Knochenplatte hindurchtritt, auf den Gewindestab 4 wird eine Mutter locker aufgeschraubt, um die Knochenplatte 2 vorläufig zu fixieren.

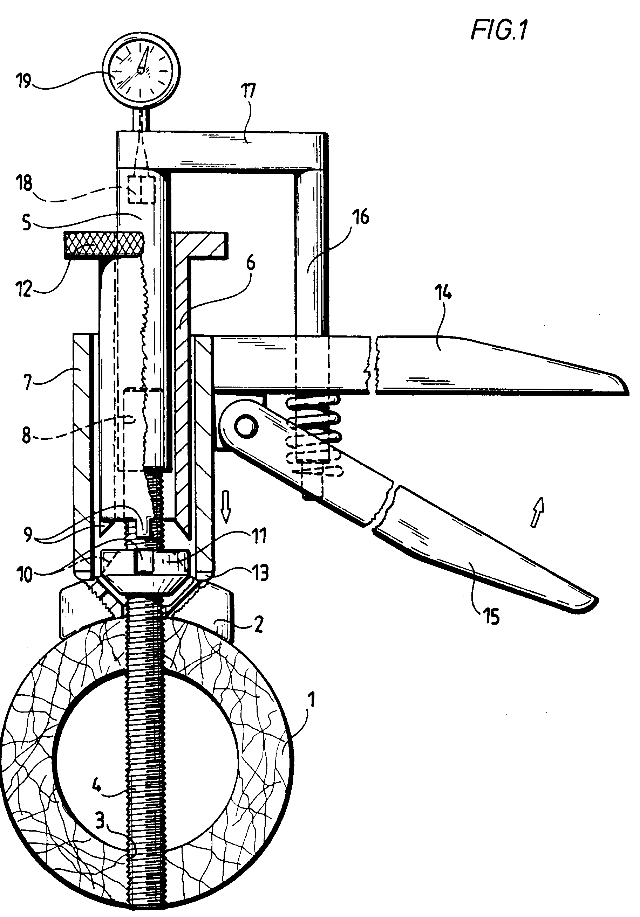

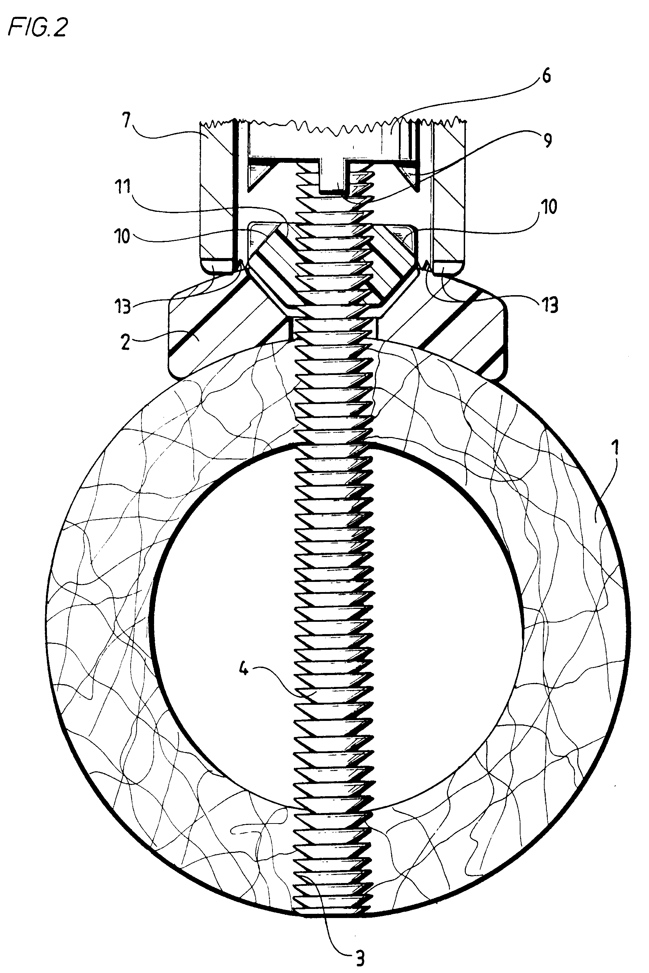

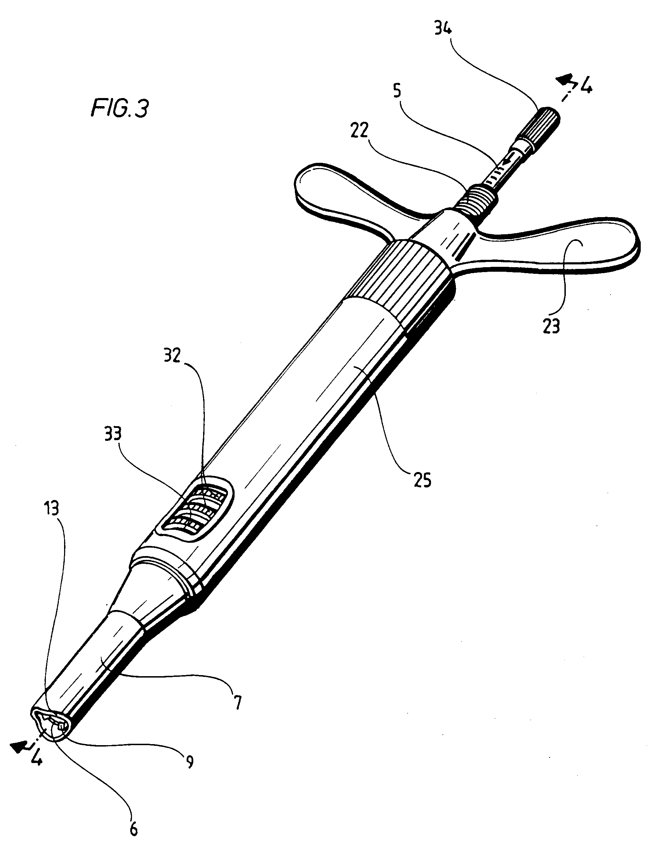

Zum Festspannen der Mutter auf dem Gewindestab wird eine Spanneinrichtung verwendet, die im wesentlichen einen zentralen Zugstab 5, ein diesen koaxial umgebendes Schraubelement 6 sowie ein dieses wiederum koaxial umgebendes Abstützelement 7 umfaßt. Der Zugstab 5 ist in dem dargestellten Ausführungsbeispiel ein massiver Stab mit einer Innengewindebohrung 8, der auf den Gewindestab 4 aufgeschraubt ist, so daß der Gewindestab mindestens 5 bis 8 mm tief in diesen eintaucht (Figuren 1 und 2).

Das Schraubelement 6 ist als Rohr ausgebildet, das den Zugstab 5 unmittelbar umgibt und an seiner unteren Kante Vorsprünge 9 trägt, die in entsprechende Ausnehmungen 10 in der Mutter 11 formschlüssig eingreifen, so daß bei einer Drehung des rohrförmigen Schraubelementes die Mutter 11 auf dem Gewindestab verdreht wird. Um diese Drehung vornehmen zu können, trägt das rohrförmige Schraubelement 6 an seinem oberen Ende eine Rändelscheibe 12. Im übrigen ist das rohrförmige Schraubelement gegenüber dem Zugstab in Längsrichtung frei verschieblich und frei verdrehbar.

Das Schraubelement 6 wird konzentrisch umgeben von dem ebenfalls rohrförmig ausgebildeten Abstützelement 7, dessen untere Kante 13, die gezahnt ist, unmittelbar auf die Knochenplatte 2 aufgesetzt wird und sich an dieser abstützt. An dem rohrförmigen Abstützelement 7 sind seitlich zwei Handgriffe 14, 15 angeordnet, einer starr und der andere verschwenkbar. Der verschwenkbare Handgriff 15 ist über eine Druckstange 16 und ein Joch 17 mit dem Zugstab 5 verbunden, wobei der Zugstab 5 gegenüber dem Joch um die Zugstablängsachse frei verdrehbar ist. Die aus den Handgriffen, der Druckstange und dem Joch bestehende Spanneinrichtung ist nur schematisch dargestellt, wesentlich ist, daß bei Betätigung der Handgriffe der Zugstab 5 nach oben gezogen wird, wobei gleichzeitig das rohrförmige Abstützelement 7 nach unten gegen die Knochenplatte 2 gedrückt wird. Als Ergebnis wird der Gewindestab 4, der in der Gewindebohrung 3 festgehalten ist, im Aufschraubbereich der Mutter 11 gespannt und geringfügig gedehnt. Dadurch ist es möglich, mittels des Schraubelementes 6 die Mutter 11 ohne größere Kraftaufwendung weiter auf den Gewindestab zu schrauben, wobei es vorteilhaft ist, das Aufschraubdrehmoment mittels eines Drehmomentbegrenzers unter einem Maximalwert zu halten. Läßt man die Spannvorrichtung wieder los, entfällt die dem Gewindestab erteilte Zugspannung, jedoch wird nunmehr der Gewindestab durch die weit herabgeschraubte Mutter 11 weiterhin unter Zugspannung gehalten, das heißt auf diese Weise wird die Knochenplatte 2 über die Mutter 11 kräftig gegen den Knochen 1 gespannt (Figur 2).

Wesentlich ist dabei, daß beim Aufschrauben der Mutter 11 keine großen Drehkräfte aufgewendet worden sind, die möglicherweise die Festigkeit des unter starker Zugspannung stehenden Gewindestabes hätte beeinträchtigen können.

In Figur 1 ist in dem Zugstab 5 ein Drucksensor 18 eingezeichnet, der beispielsweise die Form einer Feder oder eines Dehnungsmeßstreifens haben kann und in Abhängigkeit von der am Zugstab angreifenden Zugkraft auf einem Anzeigegerät 19 die jeweilige Zugkraft anzeigt. Selbstverständlich könnten diese Sensoren auch in anderen die Zugkraft übertragenden Teilen der Vorrichtung angeordnet sein, wesentlich ist lediglich die Möglichkeit, der Betätigungsperson die aufgebrachte Spannung anzuzeigen, so daß überprüft werden kann, wann die maximal mögliche Dehnung des Gewindestabes erreicht ist, die mit Sicherheit noch nicht zu einem Reißen des Gewindestabes führt.

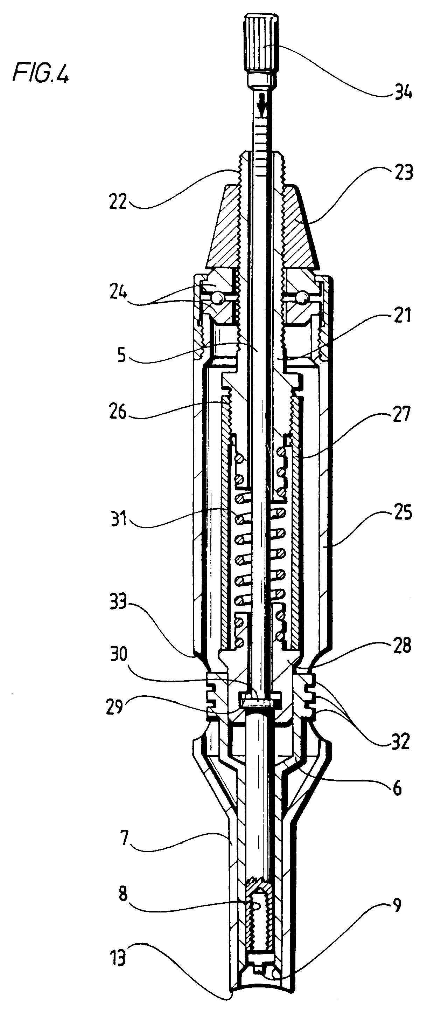

Bei einem weiteren Ausführungsbeispiel einer Spannvorrichtung, wie sie in den Figuren 3 und 4 dargestellt ist, ist prinzipiell ein ähnlicher Aufbau gewählt, einander entsprechende Teile tragen daher dieselben Bezugszeichen.

Der zentrale Zugstab 5 ist bei diesem Ausführungsbeispiel in einer Hülse 21 frei verschieblich geführt, die in ihrem oberen Bereich ein Außengewinde 22 trägt, auf welches eine Flügelmutter 23 aufgeschraubt ist. Diese stützt sich mittels eines Kugellagers 24 an einem zylindrischen Gehäuse 25 ab, welches die Spannvorrichtung umschließt und an seinem unteren Ende in das rohrförmige Abstützelement 7 übergeht.

Auf ein an der Unterseite angeordnetes Außengewinde 26 der Hülse 21 ist eine Abstandshülse 27 aufgeschraubt, die sich an einem zylindrischen Mitnehmer 28 abstützt. Dieser umgibt den Zugstab 5 konzentrisch und ist gegenüber diesem frei verdrehbar, durch einen in eine Ringausnehmung 29 eingreifenden Kragen 30 jedoch in axialer Richtung gar nicht oder nur begrenzt verschiebbar. Der Mitnehmer 28 wird durch eine an ihm angreifende, den Zugstab 5 umgebende Zugfeder 31, deren anderes Ende an der Hülse 21 festgelegt ist, in Richtung auf diese gespannt, so daß der Mitnehmer 28 unter der Vorspannkraft der Zugfeder 31 gegen das untere Ende der Abstandshülse 27 anliegt.

Das im unteren Teil den Zugstab 5 konzentrisch umgebende Schraubelement 6, das seinerseits von dem rohrförmigen Abstützelement 7 umgeben wird, umgibt den Mitnehmer 28 und trägt in dessen Bereich auf seiner Außenseite eine Rändelung 32, die durch seitliche Öffnungen 33 im Gehäuse 25 betätigbar ist.

An dem hinteren Ende trägt der Zugstab 5 ebenfalls eine Rändelung 34, die eine Verdrehung des Zugstabes 5 ermöglicht, wobei dieser Zugstab gegenüber der gesamten übrigen Vorrichtung verdrehbar ist.

Diese Spannvorrichtung wird in der gleichen Weise eingesetzt wie die anhand der Figuren 1 und 2 beschriebene Spannvorrichtung, wobei das Spannen des Gewindestabes mittels der Flügelmutter 23 erfolgt. Vorteilhaft ist bei dieser Vorrichtung, daß der Zugstab 5 isoliert gegenüber der restlichen Vorrichtung verdrehbar ist, so daß bei dieser Vorrichtung der in den Zugstab eingeschraubte Gewindestab allein durch Betätigung der Rändelung 34 eingeschraubt werden kann.

Beim Spannen des Gewindestabes wird die auf den Gewindestab ausgeübte Zugkraft über die Zugfeder 31 übertragen. Solange die Zugkraft kleiner bleibt als die von der Zugfeder 31 ausgeübte Federkraft, liegt dabei die Abstandshülse 27 am Mitnehmer 28 an, ohne daß sich die Zugfeder 31 weiter dehnt. Wird dagegen diese Zugkraft überschritten, dehnt sich die Zugfeder 31 und zwischen dem Mitnehmer 28 und der Abstandshülse 27 entsteht ein Abstand. Die Kraft der Zugfeder 31, bei welcher ein solches Abheben der Abstandshülse 27 von dem Mitnehmer 28 auftritt, kann durch Verschrauben der Abstandshülse 27 auf dem Außengewinde 26 eingestellt werden. Es ist dadurch möglich, beim anfänglichen Festlegen der Mutter mit wenigen Flügelmutterdrehungen eine Grundvorspannung zu erreichen, während beim endgültigen Spannen der Mutter die Spannungszunahme pro Flügelmutterdrehung entsprechend der Federkonstante der Zugfeder 31 geringer wird.

Besonders vorteilhaft ist bei dem Ausführungsbeispiel der Figuren 3 und 4 die konzentrische Ausgestaltung, außerdem ergibt sich bei diesem Ausführungsbeispiel eine sehr platzsparende Form.

Bei der Fixierung einer Fraktur geht der Operateur in der folgenden Weise vor: Nach der Einrichtung der Fraktur wird eine beispielsweise heiß zurechtgebogene Knochenplatte an den Knochen angelegt und durch spezielle Haltezangen fixiert. Durch eine Bohrung in der Platte wird unter Verwendung einer speziellen Bohrlehre ein Loch durch beide Corticalices des Knochens gebohrt, in die Bohrung wird ein Gewinde geschnitten und dann beginnt der Prozeß der Fixierung mit Hilfe der beschriebenen Spannvorrichtung.

Dazu steckt eine Operationsschwester den in einer Verpakkungskassette befindlichen, sterilen Gewindestab, der die Mutter bündig auf seinem Ende aufgeschraubt trägt, in den zentralen Zugstab 5 und schraubt ihn ein. Die Mutter wird dabei durch die Vorsprünge 9 ebenfalls auf dem Gewindestab verdreht, der so weit in den Zugstab eingeschraubt wird, bis er am oberen Ende des Sackloches anstößt.

Der Operateur positioniert daraufhin die Vorrichtung so über der gewindetragenden Bohrung im Knochen, daß der Gewindestab in diese eingeschraubt werden kann, beispielsweise mit Hilfe der Rändelschraube 34 bei dem in Figur 3 dargestellten Ausführungsbeispiel. Dabei wird der Gewindestab so weit eingeschraubt, bis die Mutter auf der Platte aufsitzt.

Es kann nun in der beschriebenen Weise durch Anwendung von Zug am Gewindestab und durch Festschrauben der Mutter die notwendige Spannung der Platte erreicht werden. Wesentlich ist dabei, daß für den Operateur nur wenige Handgriffe notwendig sind, da das Gerät bereits außerhalb des Operationsbereiches bestückt wird und dem Operateur somit die Möglichkeit gibt, mit Hilfe dieses Gerätes den Gewindestab einzuschrauben, die Mutter vor- und schließlich auch festzuspannen.

Vorteilhaft ist es, wenn bei der Operation unmittelbar vor dem Wundschluß nochmals die bereits fixierten Schraubverbindungen nachgespannt werden, wozu das beschriebene Gerät noch einmal aufgesetzt und zum Nachspannen verwendet wird. Damit ist es möglich, ein Kriechverhalten der beteiligten Materialien, also des Knochengewebes und der Schrauben, aufzufangen. Durch eine Temperaturerhöhung im Inneren des Körpers kann sonst durch das Kriechverhalten dieser Materialien ein Nachlassen der gewünschten Spannung auftreten, durch dieses Nachspannen vor dem Wundschluß kann mit dem beschriebenen Gerät in einfachster Weise ein solches unerwünschtes Verhalten verhindert werden. Um dies zu erreichen, genügt es, wenn der Operateur das beschriebene Gerät erneut auf den überstehenden Gewindestab aufschraubt und in der beschriebenen Weise die Mutter festdreht. Erst nach diesem Nachspannen kann gegebenenfalls der überstehende Teil des Gewindestabes entfernt werden.

## Patentansprüche

1. Vorrichtung zum Spannen chirurgischer Schraubverbindungen, bei der auf einen eingeschraubten Gewindestab (4) während dessen Zugbeanspruchung eine Mutter (11) aufschraubbar ist, gekennzeichnet durch einen zentralen Zugstab (5) für den Gewindestab (4), durch ein diesen Zugstab (5) koaxial umgebendes, in Längsrichtung gegenüber diesem verschieblich und um die Längsachse frei drehbar gehaltenes Schraubelement (6) für die auf dem Gewindestab (4) aufgeschraubte Mutter (11) und durch ein das Schraubelement (6) umgebendes, eine untere Abstützfläche (13) aufweisendes Stützelement (7), an dem eine Spannvorrichtung (14, 15) gehalten ist, die über Umlenkelemente (16, 17) an dem Zugstab (5) angreift und diesen von der Stützfläche (13) nach oben zieht.

2. Vorrichtung nach Anspruch 1, dadurch gekennzeichnet, daß der zentrale Zugstab (5) ein auf den Gewindestab (4) passendes Innengewinde (8) aufweist.

3. Vorrichtung nach Anspruch 1 oder 2, dadurch gekennzeichnet, daß das Schraubelement (6) ein den Zugstab (5) umgebendes Rohr ist.

4. Vorrichtung nach Anspruch 2 oder 3, dadurch gekennzeichnet, daß das Schraubelement (6) formschlüssig in Ausnehmungen (10) in der Mutter (11) passende Vorsprünge (9) trägt.

5. Vorrichtung nach einem der voranstehenden Ansprüche, dadurch gekennzeichnet, daß das Stützelement (7) ein das Schraubelement (6) konzentrisch umgebendes Rohr ist.

6. Vorrichtung nach einem der voranstehenden Ansprüche, dadurch gekennzeichnet, daß die Abstützfläche (13) des Stützelementes (7) eine Zahnung trägt.

7. Vorrichtung nach einem der voranstehenden Ansprüche, dadurch gekennzeichnet, daß die Spannvorrichtung zwei gegeneinander verschwenkbare Griffe (14, 15) trägt, von denen einer über eine Untersetzung seine Bewegung auf den Zugstab (5) überträgt.

8. Vorrichtung nach Anspruch 7, dadurch gekennzeichnet, daß die Spannvorrichtung einen Freilauf für ein die Spannvorrichtung betätigendes Handelement und eine Arretierung für den Zugstab (5) oder mit diesem verbundene Umlenkelemente aufweist.

9. Vorrichtung nach einem der Ansprüche 1 bis 6, dadurch gekennzeichnet, daß auf dem Zugstab (5) oder einem mit dem Zugstab (5) verbundenen Zugelement (21) eine Spannmutter (23) aufgeschraubt ist, die an der Vorrichtung frei drehbar gelagert ist und sich an dieser in axialer Richtung abstützt.

10. Vorrichtung nach Anspruch 9, dadurch gekennzeichnet, daß die Spannmutter (23) eine Flügelmutter ist.

11. Vorrichtung nach Anspruch 9 oder 10, dadurch gekennzeichnet, daß die Spannmutter (23) gegenüber der Vorrichtung mittels eines Kugellagers (24) gelagert ist.

12. Vorrichtung nach einem der voranstehenden Ansprüche, dadurch gekennzeichnet, daß am Zugstab (5) eine Zugfeder (31) angreift, die mit einem von der Spannvorrichtung verschiebbaren Zugelement (21) verbunden ist.

13. Vorrichtung nach Anspruch 12, dadurch gekennzeichnet, daß das Zugelement (21) sich an dem Zugstab (5) oder einem daran axial festgelegten Teil (28) mittels eines Abstandshalters (27) abstützt, welcher so lang ist, daß die Zugfeder (31) gespannt ist.

14. Vorrichtung nach Anspruch 13, dadurch gekennzeichnet, daß die effektive Länge des Abstandshalters (27) einstellbar ist.

15. Vorrichtung nach einem der Ansprüche 9 bis 14, dadurch gekennzeichnet, daß das Abstützelement (7) in ein die Vorrichtung umgebendes Gehäuse (25) übergeht.

16. Vorrichtung nach Anspruch 15, dadurch gekennzeichnet, daß in dem Gehäuse (25) mindestens eine Öffnung (33) zum Verdrehen des Schraubelements (6) angeordnet ist.

17. Vorrichtung nach Anspruch 16, dadurch gekennzeichnet, daß das Schraubelement (6) im Bereich der Öffnung (33) gerändelt ist.

18. Vorrichtung nach einem der voranstehenden Ansprüche, dadurch gekennzeichnet, daß der Zugstab (5) frei um seine Längsachse drehbar ist und an seinem der Verbindungsstelle zum Gewindestab (4) abgewandten Ende einen Drehgriff (34) trägt.

19. Vorrichtung nach Anspruch 18, dadurch gekennzeichnet, daß am Zugstab (5) ein Mitnehmer (28) frei drehbar und in axialer Richtung gar nicht oder beschränkt verschiebbar gelagert ist, an dem die Spannvorrichtung (14, 15; 23) angreift.

20. Vorrichtung nach einem der voranstehenden Ansprüche, dadurch gekennzeichnet, daß in dem Zugstab (5), der Spannvorrichtung oder den Umlenkelementen Druckbeziehungsweise Zugsensoren (18) angeordnet sind, die eine Anzeige (19) betätigen.

21. Vorrichtung nach Anspruch 8, dadurch gekennzeichnet, daß die Anzeige (19) erst ab einem festgelegten Minimalwert betätigbar ist.

## Claims

1. A device for tensioning surgical screw connections, wherein a nut (11) can be screwed onto a screwed-in threaded rod (4) during tension loading of the latter, characterised by a central tension rod (5) for the threaded rod (4), by a screwing element (6) for the nut (11) screwed on the threaded rod (4), the screwing element coaxially surrounding this tension rod (5) and being held so as to be displaceable in the longitudinal direction in relation to the tension rod (5) and so as to be freely rotatable about the longitudinal axis, and by a supporting element (7) which surrounds the screwing element (6) and has a lower support surface (13) and on which there is held a tensioning device (14, 15) which acts upon the tension rod (5) via deflection elements (16, 17) and draws it upwards away from the supporting surface (13).

2. A device in accordance with Claim 1, characterised in that the central tension rod (5) has an internal thread (8) matched to the threaded rod (4).

3. A device in accordance with Claim 1 or 2, characterised in that the screwing element (6) is a tube surrounding the tension rod (5).

4. A device in accordance with Claim, 2 or 3, characterised in that the screwing element (6) bears projections (9) which fit in a positive-locking manner into recesses (10) in the nut (11).

5. A device in accordance with any one of the preceding Claims, characterised in that the supporting element (7) is a tube concentrically surrounding the screwing element (6).

6. A device in accordance with any one of the preceding Claims, characterised in that the support surface (13) of the supporting element (7) bears toothing.

7. A device in accordance with any one of the preceding Claims, characterised in that the tensioning device bears two grips (14, 15) one of which is pivotable in relation to the other and transmits its movement to the tension rod (5) via a velocity ratio.

8. A device in accordance with Claim 7, characterised in that the tensioning device has an idle running device for a hand-operated element operating the tensioning device and has a stop device for the tension rod (5) or for deflection elements connected to the latter.

9. A device in accordance with any one of Claims 1 to 6, characterised in that a tensioning nut (23) is screwed on the tension rod (5) or on a tension element (21) connected to the tension rod (5), this tensioning nut (23) being mounted on the device so as to be freely rotatable and being supported on the device in the axial direction.

10. A device in accordance with, Claim 9, characterised in that the tensioning nut (23) is a wing nut.

11. A device in accordance with Claim 9 or 10, characterised in that the tensioning nut (23) is mounted relative to the device by means of a ball bearing (24).

12. A device in accordance with any one of the preceding Claims, characterised in that a tension spicing (31) acts upon the tension rod (5) and is connected to a tension element (21) displaceable by the tensioning device.

13. A device in accordance with Claim 12, characterised in that the tension element (21) is supported, by means of a spacer (27), on the tension rod (5) or on a part (28) axially secured to the latter, this spacer (27) being of such a length that the tension spring (31) is tensioned.

14. A device in accordance with Claim 13, characterised in that the effective length of the spacer (27) is adjustable.

15. A device in accordance with any one of Claims 9 to 14, characterised in that the supporting element (7) merges into a housing (25) surrounding the device.

16. A device in accordance with Claim 15, characterised in that for rotation of the screwing element (6), there is at least one opening (33) arranged in the housing (25).

17. A device in accordance with Claim 16, characterised in that the screwing element (6) is knurled in the region of the opening (33).

18. A device in accordance with, any one of the preceding Claims, characterised in that the tension rod (5) is freely rotatable about its longitudinal axis and bears a rotation grip (34) at its end facing away from the connecting site to the threaded rod (4).

19. A device in accordance with Claim 18, characterised in that a driver (28) is mounted on the tension rod (5) so as to be fixed or displaceable to a limited extent in the axial direction and freely rotatable, the tensioning device (14, 15; 23) acting upon the driver (28).

20. A device in accordance with any one of the preceding Claims, characterised in that pressure or tension sensors (18) are arranged in the tension rod (5), the tensioning device or the deflection elements and actuate a display (19).

21. A device in accordance with Claim 8, characterised in that the display (19) can only be actuated above a fixed minimum value.

## Revendications

1. Dispositif pour le serrage de liaisons vissées dans le domaine chirurgical, dans lequel un écrou (11) est vissé sur une tige filetée (4) pendant sa sollicitation par traction, caractérisé par une tige de traction centrale (5) pour la tige filetée (4), par un élément de vissage (6) tenu de façon à entourer coaxialement cette tige de traction (5), mobile en direction longitudinale par rapport à celle-ci et en rotation libre autour de l'axe longitudinal, cet élément de vissage (6) étant destiné à l'écrou (11) vissé sur la tige filetée (4), et par un élément d'appui (7), qui entoure l'élément de vissage (6) et présente une surface d'appui inférieure (13), sur lequel est tenu un dispositif de serrage (14, 15) qui s'engage via des éléments de renvoi (16, 17) dans la tige de traction (5) et qui tire celle-ci depuis la surface d'appui (13) vers le haut.

2. Dispositif selon la revendication 1, caractérisé en ce que la tige de traction centrale (5) présente un taraudage (8) adapté à la tige filetée (4).

3. Dispositif selon l'une ou l'autre des revendications 1 et 2, caractérisé en ce que l'élément de vissage (6) est un tube qui entoure la tige de traction (5).

4. Dispositif selon l'une ou l'autre des revendications 2 et 3, caractérisé en ce que l'élément de vissage (6) porte des saillies (9) ajustées en coopération de formes dans des évidements (10) dans l'écrou (11).

5. Dispositif selon l'une quelconque des revendications précédentes, caractérisé en ce que l'élément d'appui (7) est un tube qui entoure de façon concentrique l'élément de vissage (6).

6. Dispositif selon l'une quelconque des revendications précédentes, caractérisé en ce que la surface d'appui (13) de l'élément d'appui (7) porte une denture.

7. Dispositif selon l'une quelconque des revendications précédentes, caractérisé en ce que le dispositif de serrage porte deux poignées (14, 15) en pivotement l'une par rapport à l'autre, dont l'une transmet son mouvement à la tige de traction (5) via une démultiplication.

8. Dispositif selon la revendication 7, caractérisé en ce que le dispositif de serrage présente une course morte pour un élément à main qui actionne le dispositif de serrage, et un arrêt pour la tige de traction (5) ou pour des éléments de renvoi reliés à celle-ci.

9. Dispositif selon l'une quelconque des revendications 1 à 6, caractérisé en ce que sur la tige de traction (5) ou sur un élément de traction (21) relié à la tige de traction (5) est vissé un écrou de serrage (23) qui est monté en rotation libre sur le dispositif et qui s'appuie contre celui-ci en direction axiale.

10. Dispositif selon la revendication 9, caractérisé en ce que l'écrou de serrage (23) est un écrou à oreilles.

11. Dispositif selon l'une ou l'autre des revendications 9 et 10, caractérisé en ce que l'écrou de serrage (23) est monté par rapport au dispositif au moyen d'un palier à billes (24).

12. Dispositif selon l'une quelconque des revendications précédentes, caractérisé en ce qu'un ressort de traction (31) attaque la tige de traction (5), ce ressort de traction (31) étant relié à un élément de traction (21) susceptible d'être déplacé par le dispositif de serrage.

13. Dispositif selon la revendication 12, caractérisé en ce que l'élément de traction (21) s'appuie contre la tige de traction (5) ou contre une pièce (28) axialement fixée sur celle-ci, au moyen d'un élément d'écartement (27) qui est si long que le ressort de traction (31) est tendu.

14. Dispositif selon la revendication 13, caractérisé en ce que la longueur effective de l'élément d'écartement (27) est réglable.

15. Dispositif selon l'une quelconque des revendications 9 à 14, caractérisé en ce que l'élément d'appui (7) se transforme en un boîtier (25) qui entoure le dispositif.

16. Dispositif selon la revendication 15, caractérisé en ce que dans le boîtier (25) est agencée au moins une ouverture (33) pour tourner l'élément de vissage (6).

17. Dispositif selon la revendication 16, caractérisé en ce que l'élément de vissage (6) est moleté dans la région de l'ouverture (33).

18. Dispositif selon l'une quelconque des revendications précédentes, caractérisé en ce que la tige de traction (5) est en rotation libre autour de son axe longitudinal et porte une poignée rotative (34) sur son extrémité détournée de son point de liaison avec la tige filetée (4).

19. Dispositif selon la revendication 18, caractérisé en ce que sur la tige de traction (5) est monté un élément d'entraînement (28) en rotation libre et non mobile en direction axiale ou mobile en direction axiale de façon limitée, cet élément d'entraînement (28) est attaqué par le dispositif de serrage (14, 15 ; 23).

20. Dispositif selon l'une quelconque des revendications précédentes, caractérisé en ce que dans la tige de traction (5), dans le dispositif de serrage ou dans les éléments de renvoi sont agencés des détecteurs de pression ou des détecteurs de traction (18) qui actionnent un indicateur (19).

21. Dispositif selon la revendication 8, caractérisé en ce que l'indicateur (19) ne peut être actionné qu'à partir d'une valeur minimale prédéterminée.
